# EUROPEAN PATENT SPECIFICATION

(11) **EP 2 049 068 B1**
(45) Date of publication and mention of the grant of the patent: **23.11.2011**
(21) Application number: 07789585.2
(22) Date of filing: 25.07.2007
(51) Int. Cl.: A61K 8/02, A61K 8/31, A61K 8/34, A61K 8/38, A61K 8/41, A61K 8/44, A61K 8/60

(54) **TOPICAL ANTIACNE PREPARATIONS CONTAINING RETINOID (TAZAROTENE OR ADAPALENE), ANTIBIOTIC (CLINDAMYCIN PHOSPHATE) AND/OR KERATOLYTIC (MISCROSPONGED BENZOYL PEROXIDE)**
TOPISCHE ANTIAKNE-ZUBEREITUNGEN MIT RETINOID (TAZAROTEN ODER ADAPALEN), EINEM ANTIBIOTIKUM (CLINDAMYCINPHOSPHAT) UND/ODER KERATOLYTIKUM (BENZOYLPEROXID IN MICROSPONGE-FORM)
PRÉPARATIONS ANTI-ACNÉ À USAGE LOCAL CONTENANT UN RÉTINOÏDE (TAZAROTÈNE OU ADAPALÈNE), UN ANTIBIOTIQUE (CLINDAMYCINE PHOSPHATE) ET/OU UN KÉRATOLYTIQUE (MICROÉPONGES DE PEROXYDE DE BENZOYLE)

(30) Priority: 08.08.2006 MX PA06008988
(43) Date of publication of application: 22.04.2009
(73) Proprietor: Laboratorios Dermatologicos Darier, S.A. de C.V., Mexico D.F., C.P. 14000 (MX)
(72) Inventor: Ahumada Ayala, Fernando, Mexico D.F. C.P. 14000 (MX)
(74) Representative: Vossius & Partner
(86) International application number: PCT/IB2007/002195
(87) International publication number: WO 2008/017914

(56) References cited:
- WO-A-2006/048747
- US-A1- 2003 170 196
- US-A1- 2004 157 766
- US-A1- 2006 029 556
- US-A1- 2007 003 585
- JELVEHGARI ET AL: "The microsponge delivery system of benzoyl peroxide: Preparation, characterization and release studies" INTERNATIONAL JOURNAL OF PHARMACEUTICS, ELSEVIER BV, NL, vol. 308, no. 1-2, 3 February 2006 (2006-02-03), pages 124-132, XP005249243 ISSN: 0378-5173

## Description

The present invention relates to a topical preparation for treating mild to severe acne in the form of a pharmaceutical gel and cream comprising: Retinoid (Tazarotene or Adapalene), Antibiotic (Clindamycin Phosphate) and optionally Keratolytic (Benzoyl Peroxide in microsponges) as active ingredients and a carrier formulated with all the following components: Propylene glycol, Polyoxyl 35 Castor Oil or derivative, Mineral Oil, Emulsifying nonionic wax (Polawax), Glycerine, Carbomers, Disodium EDTA, Triethanolamine, Ethanol, Antioxidants and preservatives. Polysorbates can also be added.

Acne is an inflammatory disease of multifactor etiology affecting the pilosebaceous unit by the intervention of *Propionibacterium acnes.*

### Pathooenic factors

1. Increase in sebaceous secretions.
2. Ductal hyperkeratosis with pilosebaceous follicle obstruction.
3. Bacterial colonization by *P*. *acnes.*
4. Secondary inflammation.

The therapeutic applications of the product object of the present patent application, considering the combination of: Retinoid, Antibiotic and/or Keratolytic and the disease pathogeny, are the following:
1. Non inflammatory lesions:
   a. Closed comedons (microcysts).
   b. Open comedons (black spots or pimples).
2. Superficial inflammatory lesions:
   a. Papules.
   b. Pustules.
3. Deep inflammatory lesions:
   a. Nodules.
   b. Cysts.
   c. Maculae.
4. Residual lesions:
   a. Hyperpigmentation.
   b. Scars.

### Tazarotene

Tazarotene is a retinoid prodrug which turns into its active form, tazarotenic acid, through the rapid de-esterification in most of living beings.

Tazarotene has *Propionibacterium acnes* antiproliferative, differentiation normalizing and anti-inflammatory effects, considered as the base of its antiacne therapeutic activity.

The topical use of Tazarotene minimizes the systemic exposure and therefore provides a safety profile which is superior to that of orally administered retinoids. Darier 1 PCT/RCP

### Pharmacokinetics

Absorption: Less than 6.0% of the dose is absorbed during the followings 10 hours of topical administration.

Metabolism: Tazarotene is a prodrug which is metabolized on the skin and plasma to its active form Tazarotenic acid and other metabolites such as sulfoxides and sulfones.

The limited percutaneous penetration of Tazarotene favors this rapid metabolism to hydrophilic metabolites thus helping to prevent the drug build up in the body's lipophilic tissues.

Clearance: Clearance half-life for Tazarotene and its metabolites is from about 17 to 18 hours, urinary clearance being virtually complete during 2 to 3 days and in feces after 7 days. This short residence time in the body, along with the limited percutaneous penetration, create relatively low Tazarotene and Tazarotenic acid plasma levels.

Safety: Tazarotene topical administration and pharmacokinetic profile allow the existence of less systemic exposure to the drug and its metabolites and therefore less adverse effects, compared to oral retinoids.

Clinical phase I safety studies report that it does not cause sensitization, phototoxicity or photoallergicity.

Mutagenicity tests results (Ames, chromosomal aberration and micronuclei), indicate that Tazarotene is not mutagenic and has no clastogenic or chromosomal effects. Also, non-carcinogenic effects have been observed in other study with the topical application of Tazarotene to mice for 21 months.

Some adverse effects reported during Phase III clinical studies are burning sensation, skin scaling and dryness.

### Adapalene

Adapalene is a retinoid analogue which has shown to possess anti-inflammatory properties in both *in vivo* and *in vitro* inflammation models, being stable in the presence of oxygen, light and is chemically unreactive. It binds to retinoic acid specific nuclear receptors, such as tretinoin does, but differently from it, it does not bind to the linking proteins of the cytosolic receptors.

Adapalene's action mechanism consists in the normalization of follicular epithelial cells differentiation, which results in a decrease in the microcomedon formation.

Moreover, it has been proven that Adapalene is superior to other topical retinoids in standard anti-inflammatory studies, both *in vivo* as *in vitro,* by inhibiting human polymorphonuclear leukocyte chemotactic and chemokinetic responses, as well as inflammatory mediators metabolism by arachidonic acid lipooxidation.

This profile suggests that the cellular mediation inflammatory component of acne can be modified by Adapalene's action. Clinical studies have reported that no significant plasma levels were detected. Adapalene metabolism has been identified as a process involving O-demethylation, hydroxylation and conjugation.

Following topical administration of Adapalene, the presence of Adapalene compared to Tretinoin on the epidermis is of +51%, on the dermis of -37% and in receptor fluid of -71%, thus indicating that the systemic absorption is minimal and therefore facilitating its use.

### Clindamycin phosphate

Clindamycin phosphate is used for topical treatment of acne vulgaris, inflammatory or papulopustulous acne.

### Pharmacokinetics and pharmacodynamics

Clindamycin inhibits bacterial cell protein synthesis by linking to ribosome 50S subunit and suppressing protein synthesis.

*In vitro* and *in vivo* has shown activity against *Propionibacterium acnes.* Clindamycin alters the cellular surface of the bacterium, reducing the production of bacterial toxins and enzymes. Half-life of the antibiotic is roughly 2.9 hours.

Ninety percent or more clindamycin is known to bind plasma proteins. Only 10% of administered clindamycin is excreted intact in urine and in feces very minute amounts are found.

Multiple consecutive topical administrations of clindamycin phosphate at concentrations equivalent to 10 mg/ml in a water and isopropyl alcohol solution were shown to have very low clindamycin serum levels (0-3 ng) and less than 0.2% of the dosage was recovered in urine as clindamycin.

### Contraindications

Clindamycin is contraindicated in persons with a history of hypersensivity to clindamycin containing preparations or a history of regional enteritis or ulcerative colitis or also a history of antibiotic-associated colitis.

### Restrictions of use during pregnancy and lactation

Pregnancy: Its use must be under strict medical surveillance, provided that benefits overcome risks.

Lactation: Precautions are to be taken when using it in lactating women.

Pediatric: Safety and efficacy has not been assessed in pediatric populations under 12 years old.

Adverse side reactions: There may be signs of dryness, erythema, pruritus, and urent sensation on the application site.

There have been reports of diarrhea and colitis (including pseudomembranous colitis), abdominal pain, as well as gram negative folliculitis as adverse reactions in patients treated with topical clindamycin.

### Medication interactions and other kinds of interactions

Clindamycin has shown to have neuromuscular blockage properties, being able to increase the action of other neuromuscular blockers. However, it can be cautiously used in patients receiving such agents.

There are long term studies in animals without developing carcinogenesis, mutagenesis, teratogenesis effects nor on fertility.

### Microsponged benzoyl peroxide

Benzoyl peroxide is an antibacterial agent which has been shown to be effective against *Propionibacterium acnes,* an anaerobium which is found in hair follicles and comedons.

The antibacterial action of benzoyl peroxide seems to occur due to oxygen release.

Besides its bactericidal action, benzoyl peroxide has keratolytic and sebostatic effects which also contribute to its efficacy. When benzoyl peroxide penetrates the skin, it is metabolized by the amino acid cysteine into benzoic acid and an oxygen free radical.

Free radicals are produced as intermediates in metabolic systems, are very reactive and do not last long. Oxygen free radical released by benzoyl peroxide reacts with cell wall proteins of the bacterium, thus unfolding it and destroying the microorganism. Benzoyl peroxide is absorbed by the skin where it is metabolized into benzoic acid and excreted as benzoate through urine, such that at therapeutic dosages there is no plasma accumulation.

### Usage restrictions during pregnancy and lactation

Lactation: There are no data available regarding this; benzoyl peroxide should be used in these cases, only if it is absolutely necessary and with due precautions.

Side and adverse reactions: It can be a transient urent sensation, which disappears with continuous use. Contact dermatitis can appear. After 1-2 weeks usage, excessive skin dryness may occur.

Alterations in laboratory test results: There are no reports that benzoyl peroxide alters laboratory tests.

Precautions related to carcinogenesis, mutagenesis, teratogenesis and fertility effects: Benzoyl peroxide is not considered carcinogenic.

Likewise, benzoyl peroxide is not mutagenic according to results obtained by Ames test.

There are no data indicating it could affect reproduction ability.

Manifestations and overdose or accidental ingestion management: None.

Benzoyl peroxide is a highly oxidative agent and as a result it was impossible to combine it with other drugs in a single pharmaceutical form, in the present invention it is used coated (in microsponges) which prevents its reaction with the retinoids (Tazarotene or Adapalene) and with the antibiotic (Clindamycin Phosphate), plus the carrier which is completely inert or innocuous.

WO 2006/048747 A (GLENMARK PHARMACEUTICALS LTD [IN]; CHAUDHARI G N [IN]; KHACHANE V S [I) 11 May 2006 (2006-05-11) discloses (ex. 1) a topical preparation suitable for antiacne treatment (title), comprising:
- Adapalene + Clindamycin Phosphate
characterized in that the carrier comprises:
- Propylene glycol
- Carbomer
- EDTA
- preservative: methylparaben.

1. To provide society with a polydrug for every kind of acne having higher efficiency and safety, thus offering better results in less time with few adverse reactions.
2. Make a contribution to the dermatological market and particularly to the antiacne products area by displaying a topical use medicament containing Retionid, Antibiotic and optionally Keratolytic at the same time.

Currently there are no topical use antiacne medicaments on the market containing a retinoid (Tazarotene or Adapalene) and an antibiotic (Clindamycin Phosphate) combined or not with microsponged Benzoyl Peroxide.

The present stable formulation contains: Retinoid (Tazarotene or Adapalene), Antibiotic (Clindamycin Phosphate) and optionally Keratolytic (microsponged Benzoyl Peroxide) in a pharmaceutical form of a gel or cream, providing the patient with a quality, effective and safe product because the carrier containing them is completely inert or innocuous, said carrier excipients having high quality and being used within the allowed normative limits.

It contains 60-80% water, which allows to keep the skin hydrated, the excipients used in the formula being inert and innocuous.

Propylene glycol and Glycerine USP are used as moisturizing agents which are readily absorbed by the skin, thus preventing dryness thereof.

Polyoxyl 35 castor oil is an emulsifying and solubilizing agent which helps to dissolve the retinoid incorporating it to the carrier.

Carbomer or Carboxy vinyl polymer is an excellent gelling agent which allows to mix all the formula components providing a higher physical stability

Disodium EDTA is a ion sequestering agent which allows to keep stable the lattices formed in the formulation by the carbomer.

Ethanol is used as a solvent for the Retinoid.

Among the antioxidant agents there are: Butyl hydroxy toluene, Butyl hydroxy anisole, Propyl gallate and Sodium metabisulfite.

Triethanolamine is a neutralizer which binds carbopol molecules, thus facilitating gel formation and allowing to maintain formulation pH within allowed limits according to the skin pH.

Mineral oil is used as an emollient by allowing to remove dead cells on the skin and facilitating cleansing thereof.

Emulsifying anionic wax (Polawax) is an emulsifying agent containing ketostearyl alcohol, a polyoxyethylene derivative and a fatty acid ester, thus allowing to keep the emulsion stable in the case of a cream.

Among the preservatives there are: Methyl paraben, Propyl paraben and Butyl paraben, which help to prevent microbial contamination of the formulation.

### Quali-Ouantitative Formula

| Chemical Name | Function | % |
|---|---|---|
| Tazarotene or Adapalene | Retinoid (Antiinflammatory) | 0.05-0.10 |
| Clindamycin Phosphate equivalent to Clindamycin | Antibiotic | 1.00-2.00 |
| Microsponged Benzoyl Peroxide | Keratolytic | 2.00-5.00 |
| Propylene glycol | Moisturizer | 5.00-20.00 |
| Polyoxyl 35 castor oil | Emulsifier and Moisturizer | 2.00-10.00 |
| Mineral oil | Emollient | 2.00-15.00 |
| Polawax | Emulsifier | 2.00-10.00 |
| Glycerine USP | Moisturizer | 2.00-20.00 |
| Polysorbate 60 | Emulsifier | 2.00-5.00 |
| Polysorbate 80 | Emulsifier | 1.00-5.00 |
| Ethyl alcohol | Solvent | 2.00-10.00 |
| Carbomer | Gelling agent | 0.05-1.50 |
| Triethanolamine | Neutralizer | 0.10-1.00 |
| Methyl paraben base and salt | Preservative | 0.01-0.20 |
| Propyl paraben base and salt | Preservative | 0.01-0.20 |
| Butyl paraben base and salt | Preservative | 0.02-0.40 |
| Butyl hydroxy toluene | Antioxidant | 0.0075-0.10 |
| Butyl hydroxy anisole | Antioxidant | 0.005-0.02 |
| Propyl gallate | Antioxidant | 0.01-0.10 |
| Disodium EDTA | Disodium EDTA | 0.01-0.15 |

## Claims

1. Antiacne topical preparations comprising:
Retinoid (Tazarotene or Adapalene),
Antibiotic (Clindamycin Phosphate)
and optionally Keratolytic (Benzoyl Peroxide in microsponges),
**characterized in** comprising:
Polyoxyl 35 castor oil or any derivative or combination including castor oil; as emulsifier, moisturizer and/or cosolvent,
Propylene glycol and Glycerine USP as moisturizers,
Carbomers,
Ethanol,
Triethanolamine,
EDTA,
Mineral oil,
Emulsifying nonionic wax (Polawax),
antioxidants
and preservatives.

## Patentansprüche

1. Topische Antiaknepräparate, umfassend:
Retinoid (Tazaroten oder Adapalen),
Antibiotikum (Clindamycinphosphat),
und optional Keratolytikum (Benzoylperoxid in Mikroschwämmen),
**dadurch gekennzeichnet, dass** sie umfassen:
Polyoxyl 35 Rizinusöl oder ein Derivat davon oder eine Kombination enthaltend Rizinusöl; als Emulgator, Feuchthaltemittel und/oder Cosolvens,
Propylenglycol und Glycerin USP als Feuchthaltemittel,
Carbomere,
Ethanol,
Triethanolamin,
EDTA,
Mineralöl,
emulgierendes nicht-ionisches Wachs (Polawax),
Antioxidantien
und Konservierungsmittel.

## Revendications

1. Préparations topiques anti-acné comprenant :
un rétinoïde (tazarotène ou adapalène),
un antibiotique (phosphate de clindamycine)
et, éventuellement, un kératolytique (peroxyde de benzoyle incorporé à des micro-éponges),
**caractérisées en ce qu'**elles comprennent :
de l'huile de ricin de type Polyoxyl 35 ou un dérivé ou une combinaison quelconque comprenant de l'huile de ricin, comme émulsionnant, agent hydratant et/ou cosolvant,
du propylèneglycol et du glycérol USP comme agent hydratant,
des carbomères,
de l'éthanol,
de la triéthanolamine,
de l'EDTA,
une huile minérale,
une cire non ionique émulsionnante (Polawax),
des antioxydants
et des conservateurs.
